# EUROPEAN PATENT APPLICATION

(11) **EP 2 479 257 A1**
(43) Date of publication of application: **25.07.2012**
(21) Application number: 11173781.3
(22) Date of filing: 31.07.2007
(51) Int. Cl.: C12N 5/00, A61F 2/00

(54) **Use of aminosugars and chondrocytes for the treatment of cartilage and osteochondral defects and lesions**

(30) Priority: 03.08.2006 ES 200602111
(62) Divisional of application: 07786454.4
(71) Applicant: BIOIBERICA, S.A., 08029 Barcelona (ES)
(72) Inventor: Escaich Ferrer, Josep, 08021 Barcelona (ES); Ruhi Roura, Ramon, 08025 Barcelona (ES); Torrent Gibert, Ana Maria, 17458 Fornells de la Selva (ES)
(74) Representative: Sugrañes Patentes y Marcas

(57) **Abstract**

"Use of aminosugars and chondrocytes for the treatment of cartilage and osteochondral defects and lesions"

The present invention relates to the use of compositions comprising an aminosugar in combination with chondrocytes, and a biocompatible support matrix for the treatment of cartilage, and osteochondral defects or lesions.

## Description

### Technical sector of the invention

The present invention relates to the use of an aminosugar in combination with chondrocytes, and a biocompatible support matrix for the treatment of cartilage defects or lesions and osteochondral lesions.

### Background of the invention

Defects or lesions on the articular surface can be classified as chondral and osteochondral lesions or defects, depending on whether they penetrate the subchondral bone or not.

Chondral lesions, also known as cartilage lesions, have been a problem difficult to resolve for many years.

Cartilage is a specialized form of conjunctive tissue which is found in adults in the joints, ribs, ears, nasal septum and respiratory tract. The articular cartilage allows the bones to slide over one another. It also absorbs the stress caused by physical movement. Osteoarthritis is characterized by a degeneration of articular cartilage, which causes the bones to rub against one another, causing pain, swelling and loss of movement in the joint. The joint can become deformed with the passage of time.

Numerous studies have confirmed that cartilage, bones and soft tissues such as ligaments and tendons, especially in adults, have a limited regeneration capacity. In normal conditions, the renewal of the cartilage is a very slow process which consists of a constant synthesis (anabolism) and degradation (catabolism) of the components of the extracellular matrix. Chondrocytes are the cells in charge of this metabolism and, therefore, of the formation, maintenance and repair of the extracellular matrix in which they are immersed.

In 1987, the first implant of autologous chondrocytes in humans was carried out in Sweden (ACI technique), as a therapeutic alternative to other treatments to repair cartilage defects. The joint most frequently treated using this technique is the knee.

The implant of autologous chondrocytes is a technique which is performed in two stages. In a first surgical intervention, sheets of articular cartilage are extracted. This sample is cultured until reaching a sufficient number of chondrocytes for the implant. In a second surgical intervention, the damage area is carefully debrided and covered with a periosteum patch in which a small opening is left to inject the cultured chondrocytes. Once the chondrocytes have been implanted it is closed and the periosteum is sealed. Recently the use of a type I/III collagen membrane has been described instead of the periosteum (C.R. Gooding et al., The Knee, 13 (3), 203-210 (2006)).

From the first implant of autologous chondrocytes in humans, the interest for treatment or repair of cartilage defects or lesions using cells has been on the increase.

In 1994, a clinical study was published where the ACI technique had been used. Said study was performed on 23 patients with cartilage defects in the knee (M. Brittberg et al., N. Engl. J. Med., 331, 889-895 (1994)).

In numerous publications, new chondrocyte proliferation processes are described by the use of different matrices or scaffolds or by the use of alternative culture media to the conventional ones (WO 2005/082433; WO 2005/054446; K.F. Almqvist et al., Ann. Rheum. Dis., 60, 781-790 (2001)).

Bone tissue is a specialized conjunctive tissue which, as with the other conjunctive tissues, is formed by cells, fibres and fundamental substances, unlike the others, their extracellular components are calcified and turn it into a hard and firm material suitable for its support and protection function. It provides internal support to the body and offers places of insertion to the muscles and tendons which are essential for movement.

In the bones that actively grow, four types of bone cells are distinguished: osteoprogenitor cells, osteoblasts, osteocytes and osteoclasts. The osteoprogenitor cells are cells which are produced from mesenchymal cells and can proliferate and differentiate only to osteoblasts or osteoclasts. Osteoblasts are involved in bone formation.

Bone defects represent a great medical and socio-economic challenge. Currently, different types of biomaterials are being developed and applied for the reconstruction of damaged bone tissues. (U. Kneser, et al, Tissue engineering of bone: the reconstructive surgeon's point of view, J. Cell. Mol. Med. 10 (1), 7-19 (2006)).

Tendons are anatomic structures which join muscles to bones and ligaments are similar structures which join bones to other bones. They are cylindrical, elongated, structures, formed from dense conjunctive tissue and adapted to strain in one direction, with parallel collagen fibres.

The predominant cells in tendons are called tenocytes. Tenocytes have the function of maintaining the structure of the matrix through degradation and synthesis processes which comprise remodelling and may also contribute to a certain extent to the curing. Nevertheless, the tendon has a relatively low cell density and with little miotic activity, which explains the reduced renewal rate of the tissue and questions to what extent the cells can promote intrinsic curing. This means that treatment with exogenous cells may be an important strategy in tendon tissue engineering.

In 1994, preliminary research was performed with synthetic polymer fibres and tenocytes to develop implants for tendon engineering (Y. Cao et al. "Generation of neo-tendon using synthetic polymers seeded with tenocytes." Transplant. Proc. 26, 3390-3391 (1994)). Since then, various works have been published on tenocyte implants (WO 01/26667; US 2005/060033).

Tendon lesions are the most common orthopaedic lesions. For example, at least 100,000 Achilles Tendon lesions are diagnosed and treated annually in the USA (A. Praemer et al, "Musculoskeletal condition in the United States", 1st ed. American Academy of Orthopaedic Surgeons, Park Ridge, IL, 1992). It is also estimated that there are from 75,000 to 125,000 lesions of the Anterior Cruciate Ligament (ACL) each year in the USA (D.L. Butler et al, "Anterior Cruciate Ligament: Its normal response and replacement", Journal of Orthopaedic Research 7(6), 910-912 (1989) and around 300,000 were reported throughout the world. All these conditions can cause an important incapacity in the patients.

Ligaments, as with tendons and cartilaginous and bone tissues, have a limited self-repair capacity. For this reason, tissue engineering with cell-based therapy represents a field of great interest as a new generation therapy for these types of tissues. Works have recently been published regarding implants for ligaments (K. Tsuchiya et al., "Effects of cell adhesion molecules on adhesion of chondrocytes, ligament cells and mesenchymal stem cells", Materials Science and Engineering C 17, 79-82 (2001); J. A. Cooper, Jr, "Evaluation of the anterior cruciate ligament, medial collateral ligament, Achilles tendon and patellar tendon as cell sources for tissue-engineered ligament", Biomaterials 27, 2747-2754 (2006)).

The aforementioned conjunctive cartilage, bone, tendon and ligament tissues are fundamental components of the musculoskeletal system.

D-mannosamine (2-amino-2-deoxy-D-mannose) is an aminosugar which is used in the synthesis of sialic acid and derivatives.

Documents exist which mention or describe the proliferation of chondrocytes by the use of other aminosugars.

Patent application PCT WO2004/104183 claims the culture of chondrocytes in the presence of the aminosugar glucosamine.

Patent US 6,479,469 discloses the increase in proliferation of chondrocytes when an *N*-acylderivative of glucosamine is used.

Therefore, the problem to be solved by the present invention is to provide an alternative medical use of a composition comprising an aminosugar in combination with chondrocytes and a biocompatible support matrix in the treatment of cartilage defects or cartilage lesions and osteochondral lesions.

### Explanation of the invention

The present invention describes the use of chondrocytes in combination with an aminosugar selected from the group consisting of mannosamine, N-acetylmannosamine, mannosamine salts and mixtures thereof, wherein the chondrocytes are found in a biocompatible support matrix.

In a preferred embodiment, the biocompatible support matrix comprises a polymeric material and is suitable for implanting in the human or animal body.

In a more preferred embodiment, the aminosugar is mannosamine hydrochloride.

The present invention discloses the use of compositions comprising an amino sugar selected from the group consisting of mannosamine, N-acetylmannosamine, mannosamine salts and mixtures thereof, in combination with chondrocytes and a biocompatible support matrix. In a preferred embodiment, said biocompatible support matrixcomprises a polymeric material. The most preferred aminosugar is mannosamine hydrochloride.

Another aspect of the present invention is the use of the compositions as defined above for the preparation of an implant medicament for the treatment or prophylaxis of cartilage defects or lesions. In a preferred embodiment, the defects or lesions of cartilage are present in a osteoarthritic disease.

Another aspect of the present invention is the use of the compositions of the invention for the preparation of an implant medicament for the treatment of osteochondral defects or lesions.

Preferably, the aminosugars mannosamine, N-acetylmannosamine and mannosamine salts used in the present invention are of D series.

Said aminosugars may exist in anomeric forms α or β. The present invention includes the use of both individual α and β separate anomers and their mixtures.

Mannosamine salts with pharmaceutically acceptable acids include, without limit, the salts with the following acids: hydrochloric, hydroiodic, hydrobromic, phosphoric, sulphuric, methanesulfonic, acetic, formic, tartaric, maleic, citric, succinic, lactic, gluconic, pyruvic, fumaric, propionic, aspartic, glutamic, benzoic, ascorbic, glucuronic and malic acid.

Mannosamine salts can be prepared following standard aminosugar salt preparation processes. A process consists of previously obtaining the mannosamine base from mannosamine hydrochloride, to then add the corresponding acid depending on the salt one wants to obtain. Another process consists of obtaining the salts directly from the mannosamine hydrochloride using an anionic exchange resin previously conditioned with the acid that contains the anion of the salt that one wants to obtain or a metal salt of the acid. The technique of electrodialysis can also be used.

Mannosamine hydrochloride is a commercial product (NZP, New Zealand Pharmaceutical), but if desired it can be prepared by the epimerization of N-acetylglucosamine with an aqueous solution of Ca(OH)₂ followed by separation of the isomers, treatment with hydrochloric acid, decolouring and precipitation with solvents (Sugai et al., Bull. Chem. Soc. Jpn., 68, 3581-3589 (1995); Sugai et al., Tetrahedron, 53 (7), 2397-2400 (1997)).

In the present invention the term *"in vitro* process" also includes the process called ex *vivo.*

In the present invention the terms "chondroprogenitor cells" relate to cells that have already achieved partial differentiation and which have lost the pluripotential capacity of the stem cell. In other words, they are stem cells which, in their evolutionary progression, have committed to a certain cellular linage to give specific specialized cells. Thus, chondroprogenitor cells will give rise to chondrocytes.

In the present invention the term "support matrices" also includes the support scaffolds.

Preferably, the biocompatible support matrices or scaffolds used in the present invention are composed of a biodegradable material, for example polyglycolic acid, polylactic acid, cellulose, gelatine, collagen, pectin, alginate, dextrane, hyaluronic acid or derivatives thereof.

Matrices composed of non-biodegradable materials such as Teflon, polystyrene, polyacrylate or polyvinyl can also be used. In this case, if desired, it can be coated with a second material such as gelatine to increase the bonding of the cells to the polymer.

The matrix can be flexible or rigid. The sponge-type structure can also be used.

To proliferate or culture chondrocytes according to the process of the present invention, the cells are placed in contact with the aminosugar selected from the group consisting of mannosamine, N-acetylmannosamine, mannosamine salts or their mixtures. Before adding the aminosugar to them, the cells can be immersed in a culture medium which may contain nutrients, antibiotics, growth factors and differentiation or dedifferentiation inducers. Optionally, the aminosugar may form part of a culture medium which may contain nutrients, antibiotics, growth factors and differentiation or dedifferentiation inducers. Once proliferated, the cells can be implanted or, optionally, they can be transferred to a support matrix for their subsequent implanting. This is useful in the case that after the culture the cells have been differentiated, since on transferring them to a support matrix their redifferentiation and synthesis of the components of the extracellular matrix will occur.

Alternatively, the cells to proliferate can previously be transferred to a support matrix, to later add the culture medium which contains the aminosugar, so that the cells proliferate on the support matrix.

Before performing the implant, the cells of the support matrices containing the cells can be washed, eliminating the culture medium. Alternatively, compositions can be implanted which comprise the proliferated cells and the aminosugar or the proliferated cells, the support matrix and the aminosugar (mannosamine, N-acetylmannosamine, mannosamine salts or their mixtures). The compositions may further contain nutrients or other substances which form part of the culture medium.

The cells can be made to proliferate *in vitro* until a suitable cell volume has been developed, and then the cells can be implanted (with or without matrix) together with the aminosugar, so that the cells can continue proliferating *in vivo.* Alternatively, if desired, the cells can also be directly implanted, with or without matrix, and the aminosugar so that the proliferation takes place *in vivo.*

Culture and implant techniques known by persons skilled in the art of cell cultures and surgical implants can be used.

The compositions for use in the invention are an alternative to the implant of cells with or without matrix to repair and/or regenerate the damaged cartilage, bone, tendon or ligament.

The cells and the aminosugar may be present in the compositions of the invention in different concentrations.

The compositions of the invention which contain chondrocytes and/or chondroprogenitor cells (with or without matrix) and the aminosugar selected from the group consisting of mannosamine, N-acetylmannosamine, mannosamine salts and their mixtures are useful to treat cartilage defects or lesions which may be present in any joint of the body. Said defects may be the result of a traumatism, a birth defect or a disease such as osteoarthritis.

The compositions of the invention which contain chondrocytes, osteoblasts, chondroprogenitor cells and/or osteoprogenitor cells (with or without matrix) and the aminosugar selected from the group consisting of mannosamine, N-acetylmannosamine, mannosamine salts and their mixtures are useful to treat bone or osteochondral defects or lesions which may be the result of a traumatism, genetic defect or disease. They are also useful to treat bone mass losses which may arise, for example, with age, the menopause or as a consequence of diseases.

In the present invention the term "treatment" includes the terms "reparation" and "regeneration".

When in the present invention we speak of "defect" or "lesion", this also includes the terms "wear" or "loss".

An advantage of the present invention is that when using mannosamine hydrochloride the proliferation of chondrocytes is greater than when a standard culture medium is used or when the aminosugars, glucosamine hydrochloride and N-acetylglucosamine are used.

Another advantage is that when mannosamine hydrochloride reduces the release of GAGs by the chondrocytes and the metalloprotease activity in comparison with a standard culture medium.

Another important advantage of the present invention is that when N-acetylmannosamine is used in a chondrocyte culture, there is an increase in GAGs synthesis. This anabolism stimulating activity is important since the GAGs form part of the extracellular matrix.

### Brief description of the figures

Figure 1 represents at three concentrations (1, 5 and 10 mM), the effect of mannosamine hydrochloride, glucosamine hydrochloride and N-acetylglucosamine on the levels of deoxyribonucleic acid (DNA) per alginate bead.
Figure 2 represents at two concentrations (1 and 10 mM), the effect of mannosamine hydrochloride, glucosamine hydrochloride and N-acetylglucosamine on the percentage of glycosaminoglycans (GAGs) released with respect to the total GAGs by the chondrocytes in alginate beads after stimulation with IL-1β.
Figure 3 represents at two concentrations (5 and 10 mM), the effect of the mannosamine hydrochloride, glucosamine hydrochloride and N-acetylglucosamine on the metalloprotease activity (MMP) of the chondrocytes in alginate beads, after stimulation with IL-1β. and activation with APMA (4-aminophenyl mercury acetate).
Figure 4 represents at two concentrations (5 and 10 mM), the effect of the N-acetylmannosamine and N-acetylglucosamine on GAG levels with respect to levels of DNA.

### Detailed description of the preferred embodiments

The following examples are merely illustrative and do not represent a limitation of the scope of the present invention.

### Example 1: Proliferation of chondrocytes measured through DNA levels

The objective was to determine the effect of mannosamine hydrochloride on chondrocyte proliferation in an *in vitro* culture model.

The activity of mannosamine hydrochloride was compared with the following compounds: glucosamine hydrochloride and N-acetylglucosamine.

The increase in deoxyribonucleic acid (DNA) levels was determined to quantify the increase in chondrocytes.

### Materials and methods

Chondrocytes of bovine origin were isolated using digestion with collagenase following a previously described process (B. Beekman et al., Exp. Cell Res., 237, 135-141 (1997)).

The isolated bovine chondrocytes were cultured following a methodology described in the literature (J. DeGroot, et al., Exp. Cell Res., 266, 303-310 (2001)). The chondrocytes were transferred to an alginate support matrix, forming alginate beads which contain the cells at a concentration of 2.5 x 10⁶ cells/ml, and DMEM (Dulbecco's Modified Eagle's Medium) with Glutamax was added to them supplemented with ascorbic acid, penicillin, streptomycin and FCS (foetal cow serum). After 24 hours, the medium was renewed and the chondrocytes contained in alginate beads (5 beads/well, with 250 µl of medium/well, in plates of 48 wells) were cultured in the absence (control) or presence of the compound to test (at concentrations of 1, 5 and 10 mM). The medium was regenerated twice a week. At the end of 10 days, the beads were collected (5 beads in each well, n=3 wells per condition) and they were stored at a temperature of ―20°C until making the analysis.
To quantify DNA levels, the beads were digested with papain and Hoechst 33258 (bisbenzimide) dye was used. DNA levels per alginate bead were determined.
The evaluation of the differences between all compounds and the control condition was assayed using analysis of variance (ANOVA). Then, the LSD test was performed to determine the difference between each compound and the control condition. They were considered statistically significant when p ≤ 0.05.

### Results

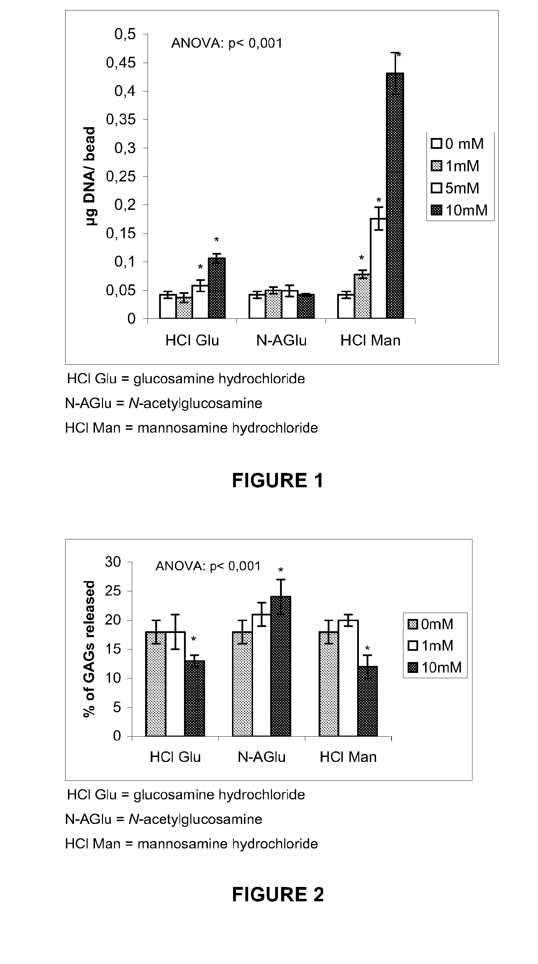

At the three concentrations tested (1, 5 and 10 mM) the highest levels of DNA/bead measured were obtained in the cells cultured with mannosamine hydrochloride (Figure 1).

Both mannosamine hydrochloride and glucosamine hydrochloride caused a dose-dependent increase in DNA levels. Significant difference was found between mannosamine hydrochloride and glucosamine hydrochloride at the three concentrations tested. At the concentration of 10 mM, mannosamine hydrochloride caused an increase 10 times higher than the control (without compound), whilst the increase caused by glucosamine hydrochloride was 4 times higher.

No significant effect on DNA content was observed with N-acetylglucosamine.

We can conclude that mannosamine hydrochloride stimulates chondrocyte proliferation, being much more effective than glucosamine hydrochloride.

### Example 2: Release of glycosaminoglycans from the chondrocytes in

### alginate beads

The objective was to determine the effect of mannosamine hydrochloride on the release of glycosaminoglycans (GAGs) from the chondrocytes in alginate beads, after stimulation with IL-1β.

GAG release is a test used to evaluate the effects of the compounds on proteoglycan degradation induced by IL-1β in the chondrocytes. It permits to evaluate the catabolic activity of the chondrocytes. The release of GAGs induced by IL-1β is mainly mediated by aggrecanases.

The activity of mannosamine hydrochloride was compared with the following compounds: glucosamine hydrochloride and N-acetylglucosamine.

### Materials and methods

Chondrocytes of bovine origin were cultured for 21 days in alginate beads as support matrix; approximately 100 beads in 75 cm² culture dishes in 25 ml of medium, without adding the compounds to be tested. The medium was regenerated twice a week. After 21 days of culture, the beads were transferred to 48-well plates, 5 beads per well, 250 µl of medium per well. The compounds to be tested were added on day 21 (mannosamine hydrochloride, glucosamine hydrochloride and N-acetylglucosamine) to evaluate the effect on the degradation of the extracellular matrix after stimulation with IL-1β (20 ng/ml). Each compound was tested at concentrations of 1 and 10 mM. In each experiment each concentration was tested at n=3 wells, except in the control (n=8 wells). Once the alginate beads have been incubated with IL-1β (20 ng/ml) and the compounds during 48 hours, the culture medium was collected (day 21 to day 23). The GAGs content in the beads and in the culture medium on day 23 was measured (release of GAGs mediated by aggrecanase). The GAGs content was determined, after digestion with papain, with the Blyscan^{TM} assay (kit) from Biocolor Ltd.

The results were expressed as a percentage of released GAGs with respect to total GAGs.

The evaluation of the differences between all compounds and the control condition was assayed using analysis of variance (ANOVA). Then, the LSD test was performed to determine the difference between each compound and the control condition. They were considered statistically significant when p **≤** 0.05.

### Results

At the upper concentration (10 mM), mannosamine hydrochloride, as with glucosamine hydrochloride, reduced the release of GAGs by the chondrocytes (Figure 2).

Mannosamine hydrochloride reduced the release of GAGs by 30% with respect to the control (culture without compound).

The release of GAGs in the osteoarthritic cartilage is greater in comparison with the normal cartilage, which entails less proteoglycan content in the affected tissue; therefore, a reduction in the release of GAGs is beneficial as it leads to a reduction in proteoglycan loss in the osteoarthritic pathology.

### Example 3: Inhibition of metalloprotease activity

The objective was to determine the effect of mannosamine hydrochloride on metalloprotease activity of the chondrocytes in alginate beads, after stimulation with IL-1β.

The metalloprotease activity is used to evaluate the effects of the compounds on the release of metalloproteases (MMPs) induced with IL-1β in the chondrocytes. It is a useful test to evaluate the catabolic activity of the chrondrocyte. Metalloproteases are secreted by the chondrocytes as inactive pro-enzymes and are previously activated using APMA (4-aminophenyl mercury acetate).

The first cause of pathological destruction of the cartilage is high proteolytic activity. Metalloproteases are a type of enzyme which degrade the extracellular matrix of the articular cartilage. These enzymes have great capacity to degrade the triple helix of the collagen. High levels of collagenases have been found in patients with osteoarthritis and also a relation between those levels and the severity of the osteoarthritic lesions.

The activity of mannosamine hydrochloride was compared with the following compounds: glucosamine hydrochloride and N-acetylglucosamine.

### Materials and methods

Bovine chondrocytes were used as starting point and the methodology of the Example 2 was followed, but in this case each compound was tested at two concentrations (5 and 10 mM).

The chondrocytes were cultured for 21 days in alginate to produce extracellular matrix. After 2 days of stimulation with IL-1β and with or without compounds, the medium was renewed. The metalloprotease activity was then determined in the culture medium after 2 hours of incubation with 20 ng/ml of IL-1β, 1 mM of APMA and with or without the compounds. The metalloprotease activity was quantified using the substrate for metalloproteases TNO 211-F (I. Tchetverikov et al., Clin. Exp. Rheumatol. 21, 711-8 (2003)).

The evaluation of the differences between all compounds and the control condition was assayed using analysis of variance (ANOVA). Then, the LSD test was performed to determine the difference between each compound and the control condition. They were considered statistically significant when p ≤ 0.05.

### Results

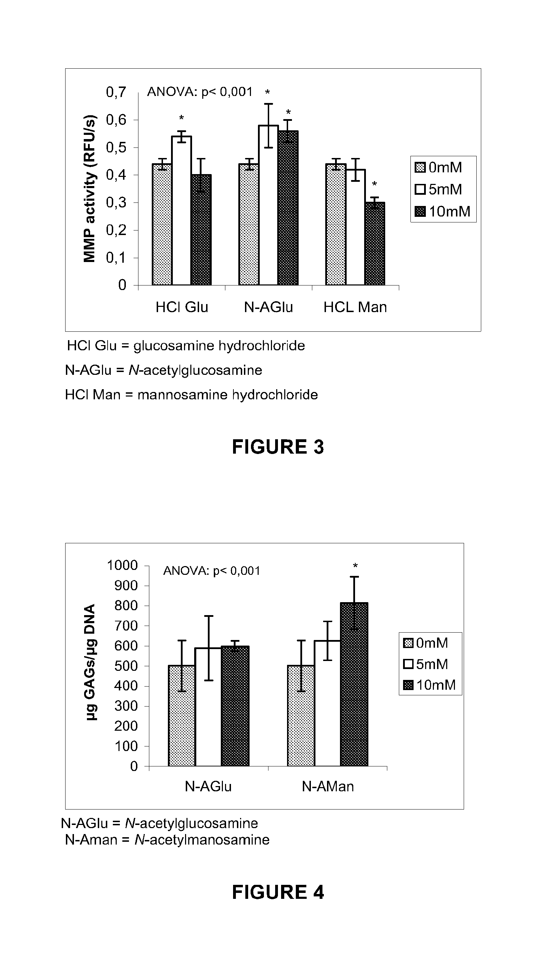

As can be observed in Figure 3, from all the compounds tested, mannosamine hydrochloride is the only one which reduced metalloprotease activity.

At a concentration of 10 mM, mannosamine hydrochloride caused a reduction in metalloprotease activity of 30% with respect to the control.

### Example 4: Determination of glycosaminoglycan content per chrondrocyte

The objective was to determine the effect of mannosamine hydrochloride on GAGs content by DNA, i.e. corrected by the number of chondrocytes.

It is an assay which permits evaluating the effects of the compounds on the deposition of GAGs per chrondrocyte, being useful to evaluate the anabolic activity of the chrondrocyte.

N-acetylmannosamine was compared with N-acetylglucosamine.

### Materials and methods

Bovine chondrocytes were used as starting point and the same methodology was followed as in Example 1 (chrondrocyte proliferation measured through DNA levels), but in this case each compound was tested at two concentrations (5 and 10 mM).

The GAG content was determined, after digestion with papain, with the Blyscan^{TM} assay (kit) from Biocolor Ltd.

The evaluation of the differences between all compounds and the control condition was assayed using analysis of variance (ANOVA). Then, the LSD test was performed to determine the difference between each compound and the control condition. They were considered statistically significant when p ≤ 0.05.

### Results

As can be observed in Figure 4, N-acetylmannosamine caused a dose-dependent increase in GAGs levels per chrondrocyte.

At the concentration of 10 mM, N-acetylmannosamine increased the GAG content per chrondrocyte by 63% with respect to the control (culture without product).

No significant effect on GAGs content per chondrocyte was observed with N-acetylglucosamine.

## Claims

1. Use of a composition comprising an aminosugar selected from the group consisting of mannosamine, N-acetylmannosamine, mannosamine salts and mixtures thereof in combination with chondrocytes and a biocompatible support matrix, for the preparation of an implant medicament for the treatment or prophylaxis of a cartilage defect or lesion or an osteochondral defect or lesion.

2. Use according to claim 1, wherein the cartilage defect or lesion is present in the osteoarthritic disease.

3. Use according to claim 1 or 2, wherein the biocompatible support matrix comprises a polymeric material.

4. Use according to any one of claims 1 to 3, wherein the biocompatible support matrix is an alginate support matrix.

5. Use according to any one of claims 1 to 4, wherein the aminosugar is mannosamine hydrochloride.
